Europäisches Patentamt

European Patent Office

Office européen des brevets

(11) Publication number: **0 210 989**
**B1**

# EUROPEAN PATENT SPECIFICATION

(51) Int. Cl.⁴: **A 61 B 17/36**

(54) METHOD AND APPARATUS FOR DISTINGUISHING ORGANIC TISSUES FROM EACH OTHER AND SEPARATING THEREOF BY OPERATION.

(30) Priority: 21.03.84 HU 113084

(43) Date of publication of application:
25.02.87 Bulletin 87/09

(45) Publication of the grant of the patent:
13.09.89 Bulletin 89/37

(84) Designated Contracting States:
AT BE CH DE FR GB LI LU NL SE

(56) References cited:
WO-A-85/00010
DE-A-2 829 516
DE-A-3 306 981
US-A-4 120 293
US-A-4 266 549

(73) Proprietor: KOVACS, Adam
Buza u 2
H-2097 Pilisborosjenö (HU)

(72) Inventor: KOVACS, Adam
Buza u 2
H-2097 Pilisborosjenö (HU)

(74) Representative: Senior, Alan Murray et al
J.A. KEMP & CO 14 South Square Gray's Inn
London WC1R 5EU (GB)

Courier Press, Leamington Spa, England.

## Description

### Technical field

The invention relates to a method and apparatus for distinguishing organic tissues from each other and separating thereof by operation.

### Background art

In the therapy and even in the surgery laser knives of different type are used more and more wide-spread. Laser knives are described in the US—PS Nos. 4 396 285 and 4 408 602. In the US—PS No. 4 316 467 a laser knife apparatus is described where the laser beam is directed to the area to be operated with fibre optic bundle and at the same time the area, which has been already operated, is monitored by an optoreceiver through another fibre optic bundle, and the intensity of the laser beam is controlled depending on the colour of the area operated.

The DE—PS No. 26 20 846 describes a laser therapy apparatus where the laser beam is moved in three directions of the space with the aid of a regulating circuit controlled by a space sensing device. The apparatus is used also for removing carcinomas by operation, and the apparatus corrects the unintentional moving of the patient.

The chances for recovery after the removal of malignant tumours by operation is considerably influenced by the circumstance whether the malignant tissues could be removed completely. The operation is generally rendered more difficult thereby that the healthy tissues may also be injured which occasionally may cause irreparable damages. The operations performed with laser knives constitute a quality change in the precise removal of malignant tumours, with the known means and methods, however, the diseased and healthy tissues could not be distinguished from each other either. So the result of the operation was not restricted by the precise tracking of the laser knife but by the precise determination of the boundary of the diseased and healthy organ.

US—A—4120293 discloses apparatus which uses a laser to remove carbon deposits from the lungs of living animals. The apparatus may also be used for diagnosis by introducing particles on to the lung surface which are then vapourized by the laser and produce photons which are detected by an optical fibre positioned near the tissue and analysed to give a line spectrum.

Recently analytical pyrolysis, which is used widespread in the analytical chemistry, is applied for the examination of different organic tissues. According to that substances having high molecular mass are decomposed thermally and the pyrolysate so obtained is analysed. The pyrolysis may be carried out suitable by thermal decomposition or pyrolysis performed with laser beam while examining substances of low molecular weight. The organic substance is pyrolysed by a laser beam of well-defined pulse power and so pyrolysis products (gases and/or vapours) are set free. Several analytical methods are known for the analytical pyrolysis. Such are the pyrolysis gas chromatography (PY-GB), the pyrolysis gas chromatography mass spectrography (PY-GB-MS) and the pyrolysis mass spectrometry (PY-MS). From among these methods the mass spectrometry is the faster one and has the further advantage that the results of the analysis may be obtained simply also in form of digital information and so are suitable for computer processing.

There are also mass spectrometers known, the test results of which are processed by computers. Such apparatus is manufactured by Finnigan MAT GmbH in Germany of the type ITD 700 (Ion Trap Detector). This equipment produces the mass spectra characterizing for the chemical composition of the substance examined together with an IBM personal computer which is called as "dactylogram" in figurative sense since the spectrum identifies a substance unambiguously.

Further known equipment is that of Hewlett-Packard of the type of 5970A which is a mass selective detector and is connected with a gas chromatograph and a controlling and evaluating microcomputer of the type HP 9825B.

The dactylogram technique is a method according to which the spectrum of an examined and analysed substance is compared with the spectrum dactylogram of a known substance examined previously. In such cases the spectrum itself should not be analysed, only the identity or the difference is determined.

The dactylograms of the mass spectrometry pyrolysis [pyrograms] may be used very well for characterizing complex substances, biopolymers and even cells and tissues. Such examinations are described in the Analytical and Applied Pyrolysis [Elsevier, Amsterdam] by M. Jarman in "DNS-pyrolysis" [Analytical and Applied Pyrolysis, *2*, 217—223 [1980]] by S. M. Huff et al. in "Pyrolysis examination of human leukemic and normal leukocytes" [Analytical and Applied Pyrolysis, *3*, 95—109 and 111—129[1981]], by W. Windig et al. in "Pyrolysis examination of frog cells and tissues" [Analitical and Applied Pyrolysis, *5*, 349—362 [1983]], as well as in Biomedical Mass Spectrometry [Heyden and Son, London] in "Pyrolysis GC/MS examination of different human cells" [Biomedical Mass Spectrometry, *6* 491—498 [1979]].

It has been found that the composition of the pyrolysate [gas and/or vapour] set free on the effect of the laser knife is unambiguously characterizing thereof whether the tissue operated is tumourous or healthy. This is based chemically thereon that the healthy and tumourous tissues are of different protein [amino acid] composition and so its pyrolysis performed under the same conditions results in different pyrolysis products. These pyrolysis products may be distinguished from each other by the dactylogram technique, i.e. according to the mass spectra.

The present invention provides apparatus comprising a laser connected to a control apparatus for vapourizing material onto which the laser beam is directed and sensor means adjacent the said material for sensing the vapourized material, and including a real time spectrum analyser, characterized in that the apparatus is adapted for distinguishing organic tissues from each other and separating them by operation, the sensor means including a pyrolysate collecting and dispatching head connected via piping with the analyser, the analyser being connected with an input of a comparing and evaluating unit which has a reference data memory connected to another input thereof, the output of the comparing and evaluating unit being connected to a display. According to a preferred embodiment of the above apparatus the display is a video display to which a video system, as well as a video camera directed to the tissue area examined are connected. The apparatus of the invention comprises preferably a computer controlling the operation of any individual units, to the inputs of which the analyser, the comparing and evaluating unit and the video system, to the outputs thereof the video display and the control apparatus of the laser knife are connected.

So that the pyrolysate examined should be analysed always under the same conditions, preferably there are a pressure sensing element and a temperature sensing element in the pyrolysate collectung and dispatching head, which are connected to the inputs of a computer, as well as said head comprises a pressure and a temperature controlling unit which are connected to the outputs of the computer.

The control apparatus of the laser knife comprises preferably a micromanipulator driving the laser knife, the control input of which is connected to a computer or a hand control apparatus.

The analyser used in the apparatus of the invention is preferably a dynamic mass spectrometer.

Thus with the present invention organic tissues, first of all the healthy and diseased tissues may be distinguished from each other during an operation simultaneously and continuously with the operation and the removal of the diseased tissues may be performed more precisely and reliably.

Thus with the apparatus, samples are taken from the tissues in such way that certain parts thereof are pyrolysed punctiform and the pyrolysate (gases and/or vapours) so obtained is collected and its composition is examined and the results of the composition examination are compared with reference data and the character of the tissue is determined, whereafter samples are taken repeatedly and the boundary of the tissues is indicated and the separation of the tissues is performed along this boundary. With the illustrated embodiment the punctiform pyrolysis is performed by laser pyrolysis and the separation of the tissues along the boundary indicated is carried out also by laser pyrolysis.

The invention will be further described by way of non-limitative example with reference to the sole accompanying Figure.

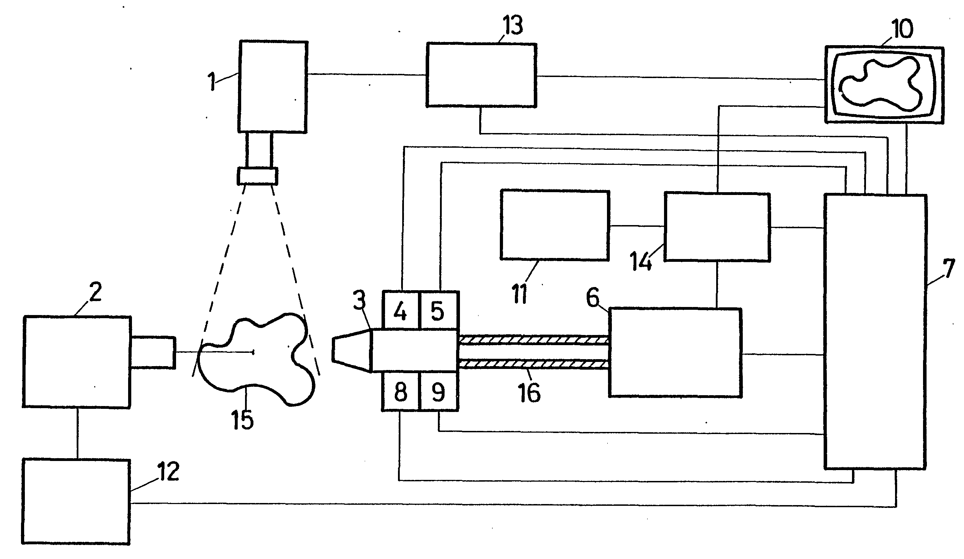

In the Figure the principled structure of the apparatus of the invention may be seen.

According to the Figure laser knife 2 is directed to the examined tissue 15, said laser knife 2 being connected to control apparatus 12. The pyrolysate collecting and dispatching head 3 is placed in the immediate vicinity of the point irradiated by the laser knife 2. The head 3 is connected preferably through capillary piping 16 to the real time analyser 6. The reference dactylograms, i.e. the mass spectra relating to the known tissue structures are stored in reference data memory 11. The analyser 6 and the data memory 11 are connected to each input of comparing and evaluating unit 14. The output of the comparing and evaluating unit is connected to display 10.

The apparatus of the invention operates in this simplest constitution as follows:

The laser knife 2 emits a laser beam to the area determined by the control apparatus 12 and this area of the examined tissue 15 is pyrolysed and vapours and/or gases are set free. The collecting and dispatching head 3 being in the immediate vicinity of the pyrolysed area of the examined tissue 15 absorbs said pyrolysates and dispatches them through the piping 16 into the analyser 6. The analyser 6 which is preferably a dynamic mass spectrometer, analyses the pyrolysates introduced and produces the result in form of digital electric signs. These digital signs get into the comparing and evaluating unit 14. In the comparing and evaluating unit 14 the reference dactylograms, i.e. the mass spectra of the pyrolysates of known tissue pieces stored in the data memory 11 are available also in form of digital signs.

On the basis of that the comparing and evaluating unit 14 compares the two mass spectra and dispatches the result, i.e. whether the pyrolysed piece is healthy or diseased, into the display 10 which indicates the result for the examining physician or operating surgeon. The laser knife 2 may be directed correspondingly through the control apparatus 12 and the examination may be performed on other places.

As laser knife 2 one can use an above mentioned known apparatus. The analyser 6 is also known per se.

A more complete embodiment of the apparatus of the invention comprises also a video apparatus which consists of a video camera 1, a video system 13 connected to the output of said video camera and a display 10 which is a video display in this case.

The camera 1 directed to the examined tissue piece 15 projects said tissue piece which will be visible faithfully on the video display 10. By simultaneous use of a computer 7 the point, to which the laser knife 2 is directed is indicated-—perhaps by a luminous point of different colour—by the video system 13 on the display 10 with the aid of coordinate information obtained from the control apparatus 12. The comparing and evaluating unit 14 connected with the computer 7 gives the video system 13 the examination result of the point marked and the video system 13 indicates the condition of the examined tissue piece on display 10 in a colour depending on the result of the examination.

These examination points are stored in the video system 13 and so the healthy and diseased areas may be marked — by different colours — on display 10.

By using the computer 7 the automatic driving of the laser knife 2 through the control apparatus 12 may be achieved ensuring that the laser knife 2 should turn away from the healthy area and only the diseased should be pyrolysed whereby the diseased tissue piece is removed. In this way it may be ensured optimally that the healthy tissue pieces remain unwounded while the diseased tissue pieces are pyrolysed possible completely or are separated along the marked boundary.

The laser knife 2 may be driven by a micromanipulator known per se or a known three dimensional driver mentioned above. The control apparatus 12 may be controlled by the computer 7 but also hand control may be applied when the physician decides about the subsequent driving of the laser knife 2 in view of the picture obtained on display 10.

**Claims**

1. Apparatus comprising a laser (2) connected to a control apparatus (12) for vapourizing material (15) onto which the laser beam is directed and sensor means (3) adjacent the said material (15) for sensing the vapourized material, and including a real time spectrum analyser (6), characterized in that the apparatus is adapted for distinguishing organic tissues from each other and separating them by operation, the sensor means (3) including a pyrolysate collecting and dispatching head (3) connected via piping (16) with the analyser (6), the analyser (6) being connected with an input of a comparing and evaluating unit (14) which has a reference data memory (11) connected to another input thereof, the output of the comparing and evaluating unit (14) being connected to a display (10).

2. The apparatus as claimed in Claim 1, characterized in that the display (10) is a video display to which a video system (13) and a video camera (1) directed to the examined tissue piece (15) are connected.

3. The apparatus as claimed in Claim 2, characterized in that a control computer (7) controls the operation of each unit, to the inputs of which the spectrum analyser (6), the comparing and evaluating unit (14) and the video system (13), to the output thereof the video display (10) and the control apparatus (12) of the laser (2) are connected.

4. The apparatus as claimed in Claim 3, characterized in that the pyrolysate collecting and dispatching head (3) contains a pressure sensing element (8) and a temperature sensing element (9) connected respectively to the inputs of the computer (7) and having also pressure and temperature controlling units (4 and 5) which are connected to the outputs of computer (7).

5. The apparatus as claimed in Claim 3 or 4, characterized in that the control apparatus (12) contains a micromanipulator driving the laser (2), the control input of which being connected with the computer (7) or a hand control apparatus.

6. The apparatus of any preceding claim, characterized in that the spectrum analyser (6) is a dynamic mass spectrometer.

**Patentansprüche**

1. Gerät, welches einen mit einer Kontrollvorrichtung (12) verbunden Laser (2) zur Verdampfung von Material (15), auf das der Laserstrahl gerichtet ist, und eine an das Material (15) angrenzende Sensoreinrichtung (3) zum Nachweis des verdampften Materials umfaßt und einen Echtzeit-Spektralanalysator (6) einschließt, dadurch gekennzeichnet, daß das Gerät zur Unterscheidung von organischen Geweben voneinander und zu ihrer operativen Trennung voneinander ausgelegt ist, wobei der Sensor (3) einen Pyrolysat sammelnden und weiterlietenden Kopf (3), der über eine Rohrverbindung (16) mit dem Analysator (6) verbunden ist, einschließt, der Analysator (6) über einen Eingang mit einer Vergleichs- und Bewertungseinheit (14) verbunden ist, die über einen anderen ihrer Eingänge mit einem Vergleichsdatenspeicher (11) verbunden ist, und der Ausgang der Vergleichs- und Bewertungseinheit (14) mit einer Anzeige (10) verbunden ist.

2. Gerät, wie in Anspruch 1 beansprucht, dadurch gekennzeichnet, daß die Anzeige (10) eine Videoanzeige ist, mit der ein Videosystem (13) und eine auf das untersuchte Gewebestück (15) gerichtete Videokamera (1) verbunden sind.

3. Gerät, wie in Anspruch 2 beansprucht, dadurch gekennzeichnet, daß ein Kontrollrechner (7) den Betrieb einer jeden Einheit überwacht, mit dessen Eingängen der Spektralanalysator (6), die Vergleichs- und Bewertungseinheit (14) und das Videosystem (13) mit dessen Ausgang die Videoanzeige (10) und die Steuervorrichtung (12) des Lasers (2) verbunden sind.

4. Gerät, wie in Anspruch 3 beansprucht, dadurch gekennzeichnet, daß der Pyrolysat sammelnde und weiterleitende Kopf (3) ein Druckfühlerelement (8) und ein Temperaturfühlerelement (9) aufweist, die jeweils mit den Eingängen des Rechners (7) verbunden sind, und weiterhin druck- und temperatursteuernde Einheiten (4) und (5) aufweist, die mit den Ausgängen des Rechners (7) verbunden sind.

5. Gerät, wie in Anspruch 3 oder 4 beansprucht, dadurch gekennzeichnet, daß das Steuergerät (12) einen Mikromanipulator enthält, der den Laser (2) führt und dessen Steuereingang mit dem Rechner (7) oder einer Handsteuervorrichtung verbunden ist.

6. Gerät nach einem der vorstehenden Ansprüche, dadurch gekennzeichnet, daß der Spektralanalysator (6) ein dynamisches Massenspektrometer ist.

**Revendications**

1. Dispositif comportant un laser (2), relié à un appareil (12) de commande, destiné à vaporiser une matière (15) sur laquelle est dirigé le rayon laser et des moyens (3) de détection situés à proximité de ladite matière (15) destinés à détecter la matière vaporisée, et comprenant un appareil (6) d'analyse spectrale en temps réel, caractérisé en ce que le dispositif est approprié pour distinguer les tissus organiques les uns des autres et les séparer par opération, les moyens (3) de détection comprenant une tête (3) de collecte et d'expédition des produits de la pyrolyse reliée par des tubulures (16) à l'appareil (6) d'analyse, l'appareil (6) d'analyse étant relié à une entrée d'une unité (14) de comparaison et d'évaluation qui possède une mémoire (11) de données de référence reliée à une autre entrée de cette unité, la sortie de cette unité (14) de comparaison et d'évaluation étant reliée à un dispositif de visualisation (10).

2. Dispositif selon la revendication 1, caractérisé en ce que le dispositif de visualisation (10) est un écran vidéo auquel sont reliés un système vidéo (13) et une caméra vidéo (1) dirigée vers le morceau (15) de tissu examiné.

3. Dispositif selon la revendication 2, caractérisé en ce qu'un ordinateur (7) de commande règle le fonctionnement de chaque unité, ordinateur aux entrées duquel sont reliés l'appareil (6) d'analyse spectrale, l'unité (14) de comparaison et d'évaluation et le système vidéo (13), et aux sorties duquel sont reliés l'écran vidéo (10) et l'appareil (12) de commande du laser (2).

4. Dispositif selon la revendication 3, caractérisé en ce que la tête (3) de collecte et d'expédition des produits de la pyrolyse contient un élément (8) détecteur de pression et un élément (9) détecteur de température reliés respectivement aux entrées de l'ordinateur (7) et comportant aussi des unités (4 et 5) de commande de la pression et de la température qui sont reliées aux sorties de l'ordinateur (7).

5. Dispositif selon la revendication 3 ou la revendication 4, caractérisé en ce que l'appareil (12) de commande contient un micromanipulateur entraînant le laser (2), micromanipulateur dont l'entrée de commande est reliée à l'ordinateur (7) ou à un dispositif de commande manuelle.

6. Dispositif selon l'une quelconque des revendications précédentes, caractérisé en ce que l'appareil (6) d'analyse spectrale est un spectromètre dynamique de masse.